(19) **Europäisches Patentamt European Patent Office Office européen des brevets**

(11) **EP 2 682 736 B1**

(12) **FASCICULE DE BREVET EUROPEEN**

(45) Date de publication et mention
de la délivrance du brevet:
**24.07.2019 Bulletin 2019/30**

(51) Int Cl.:
***G01N 15/08*** (2006.01)

(21) Numéro de dépôt: **13176044.9**

(22) Date de dépôt: **05.02.2007**

(54) **Procédé et dispositif de mesure de perméation**

Verfahren und Vorrichtung zur Durchlässigkeitsmessung

Method and device for measuring permeation

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **15.02.2006 FR 0601320**
**11.04.2006 US 402230**

(43) Date de publication de la demande:
**08.01.2014 Bulletin 2014/02**

(62) Numéro(s) de document de la (des) demande(s)
initiale(s) en application de l'article 76 CBE:
**07101753.7 / 1 821 093**

(73) Titulaire: **COMMISSARIAT À L'ÉNERGIE ATOMIQUE ET AUX ÉNERGIES ALTERNATIVES**
**75015 Paris (FR)**

(72) Inventeurs:
• **Firon, Muriel**
**91520 EGLY (FR)**
• **Cros, Stéphane**
**73000 CHAMBERY (FR)**
• **Trouslard, Philippe**
**78460 CHEVREUSE (FR)**

(74) Mandataire: **Regimbeau**
**87 rue de Sèze**
**69477 Lyon Cedex 06 (FR)**

(56) Documents cités:
**WO-A1-02/33378          WO-A2-02/088657**
**JP-A- S63 172 942       US-A1- 2003 001 086**
**US-A1- 2003 074 954     US-A1- 2004 040 372**
**US-A1- 2004 123 646**

**Description**

DOMAINE DE L'INVENTION

La présente invention concerne le domaine de la perméation de gaz, et plus particulièrement un dispositif et une méthode de mesure de perméation de gaz à travers des matériaux solides.

ETAT DE LA TECHNIQUE

La sélection de matériaux pour certaines applications, telles que le conditionnement ou l'électronique par exemple, nécessite l'étude de la perméation de certains gaz à travers ces matériaux. Par perméation, on entend le mécanisme de passage d'un gaz à travers un matériau suivant les étapes d'absorption du gaz dans le matériau, de diffusion de ce gaz à travers le matériau, et de désorption du gaz de l'autre côté du matériau. Par exemple, les mesures de perméation de gaz comme l'oxygène ou la vapeur d'eau, à travers les matériaux à sélectionner, sont les plus répandues.

Dans le cas des matériaux destinés au conditionnement agroalimentaire par exemple, l'étude de la perméation des gaz courants à travers les matériaux, et plus particulièrement de l'oxygène et de la vapeur d'eau, sont critiques. Les niveaux requis de perméabilité à ces gaz sont extrêmement faibles, et l'étude de la perméation nécessite par conséquent des dispositifs de mesure de perméation présentant des sensibilités importantes.

En réponse à cette problématique, de nombreux dispositifs de mesure de perméation ont été développés, reposant sur divers principes de suivi de gaz, et présentant chacun leurs inconvénients.

Il a été en particulier développé un dispositif de mesure des flux de perméation d'$O_2$, Ar, ou He, comprenant une enceinte de mesure sous ultravide. La sensibilité de cette méthode de mesure est néanmoins limitée par le niveau de pollution résiduelle du gaz suivi dans l'enceinte de mesure sous vide, l'oxygène et la vapeur d'eau présents dans l'atmosphère étant naturellement parmi les polluants les plus importants, même dans une enceinte sous vide.

Pour remédier à cet inconvénient majeur, il a été proposé d'utiliser, pour un gaz cible dont on cherche à connaître la perméation à travers le matériau, un gaz isotopique de ce gaz cible, c'est-à-dire ayant un nombre de masse différent. Ainsi, en utilisant par exemple un spectromètre de masse comme analyseur dans l'enceinte de mesure, il est possible de réduire les seuils de détection de la perméation de plusieurs ordres de grandeur. En effet, l'abondance isotopique naturelle de ces éléments étant très faible, la pollution de l'enceinte par ces espèces est d'autant moins importante.

Cette dernière méthode de mesure permet d'augmenter la sensibilité des mesures de perméation. Néanmoins, les temps de mesures pour des gaz cibles tels que la vapeur d'eau ou l'oxygène ne sont nullement améliorés au regard des méthodes classiques. En effet, ces méthodes ne permettent de mesurer que la perméabilité d'un gaz cible à travers un film. Les films étant très fragiles, notamment ceux comportant un revêtement en couche mince, toute manipulation risque de provoquer une altération des propriétés de barrière du film (par exemple par une rayure). Le changement de gaz cible, s'il implique une modification du dispositif instrumental, conduit souvent à changer de film pour chaque mesure, d'où un manque de reproductibilité et de fiabilité des mesures.

Un but de la présente invention est donc de proposer un dispositif et un procédé de mesure de perméation amélioré, alliant notamment sensibilité et gain en temps de mesure.

EXPOSE DE L'INVENTION

A cet effet, on prévoit selon l'invention un procédé de mesure de perméation de gaz à travers un matériau, défini par la revendication 1.

La formation préalable d'un mélange de gaz dans l'enceinte de mélange va permettre de remplir la première chambre avec un mélange comprenant éventuellement plusieurs gaz isotopiques, correspondant à autant de gaz cibles dont on cherche à étudier la perméation à travers le matériau. Ainsi, tous les gaz isotopiques du mélange vont traverser le matériau et le moyen d'analyse va donc également pouvoir analyser de manière simultanée les gaz ayant traversé.

Le premier avantage conféré par cette simultanéité des analyses réside dans les temps de mesure qui sont réduits par autant qu'il y a de gaz à analyser. Cette considération prend toute sa valeur quand on sait que la mesure de la perméation de l'$O_2$ par exemple à travers un matériau peut prendre plusieurs jours, voire mois, selon la perméabilité du matériau.

De plus, cette simultanéité des mesures permet de mettre en évidence une éventuelle interdépendance entre l'adsorption notamment de vapeur d'eau par un film et la perméabilité de ce film à un ou plusieurs autres gaz. En effet, l'adsorption de vapeur d'eau est susceptible de modifier les propriétés de diffusion d'un autre gaz, par exemple l'oxygène, à travers un film. On ne se contente plus de fixer le taux d'hygrométrie durant des mesures de perméation à l'oxygène, mais on effectue des mesures simultanées de perméation à la vapeur d'eau et à l'oxygène.

En outre, les conditions expérimentales dans lesquelles les mesures sont effectuées (notamment en termes de température, dégradation du matériau, etc.) sont identiques pour chaque gaz puisque les analyses sont faites en parallèle, et rend donc les analyses de perméation pour un matériau particulier beaucoup plus fiables.

Des aspects préférés mais non limitatifs du procédé selon l'invention sont les suivants :

- l'étape de formation du mélange de gaz isotopiques consiste à remplir l'enceinte de mélange avec au moins un gaz isotopique provenant d'une source de gaz, à fermer l'enceinte de mélange, à réguler le mélange en vapeur d'eau deutérée par un moyen de régulation de phase, et à équilibrer le mélange ;
- on remplit l'enceinte de mélange avec un gaz isotopique choisi parmi $^{18}O_2$ et $^{17}O_2$ lorsque l'un des gaz cibles est $^{16}O_2$ ;
- l'étape de remplissage de la première chambre consiste à faire le vide dans la première chambre avec un premier moyen de pompe, puis à ouvrir l'enceinte de mélange pour remplir la première chambre avec le mélange de gaz isotopiques ;
- le procédé comprend en outre une étape de régulation thermique préalable à l'étape d'analyse, l'étape de régulation thermique consistant à réguler la température de l'enceinte de perméation et de l'enceinte de mélange avec un moyen de régulation thermique ;
- l'étape d'analyse consiste à déterminer simultanément la présence de gaz isotopiques dans la deuxième chambre avec un analyseur de gaz placé dans une enceinte de mesure, l'enceinte de mesure étant reliée à la deuxième chambre et mise sous vide continu avec un deuxième moyen de pompe ;
- le procédé comprend en outre une étape préalable à l'étape d'analyse consistant à mettre sous vide la deuxième chambre et l'enceinte de mesure avec le deuxième moyen de pompe ;
- le procédé comprend en outre une étape de récupération des gaz consistant à, une fois l'analyse de perméation terminée, récupérer le mélange de gaz de la première chambre et l'acheminer vers un moyen de filtrage pour assécher les gaz du mélange ;
- l'étape de récupération des gaz comprend en outre une étape consistant à piéger les gaz asséchés du mélange dans la source de gaz correspondante avec un moyen de récupération du gaz considéré ;
- le procédé selon l'invention comprend étape préalable de test, consistant remplir la première chambre avec de l'hélium, et à calculer la perméation du matériau à l'hélium, pour effectuer une présélection du matériau en excluant le matériau des analyses ultérieures si la perméation du matériau à l'hélium est supérieure à un seuil déterminé.

On prévoit également selon un exemple ne faisant pas partie de l'invention un dispositif de mesure de perméation de gaz à travers un matériau, comprenant :

▪ Une enceinte de perméation comprenant une première et une deuxième chambre séparées par le matériau,
▪ Un moyen d'analyse de gaz pour analyser les gaz ayant traversé le matériau par perméation et étant présents dans la deuxième chambre,

caractérisé en ce qu'il comprend en outre une enceinte de mélange pour former un mélange de gaz isotopiques, chaque gaz isotopique correspondant, avec un nombre de masse différent, à un gaz cible dont on cherche à connaître la perméation à travers le matériau, l'enceinte de mélange étant reliée à la première chambre pour remplir la première chambre avec le mélange de gaz isotopiques, de sorte que le moyen d'analyse calcule simultanément la perméation du matériau à chacun des gaz cibles correspondants.

Des aspects préférés mais non limitatifs de ce dispositif sont les suivants :

- le dispositif comprend en outre une ligne d'alimentation en gaz comprenant une première et une deuxième extrémité, la première extrémité étant reliée à au moins une source de gaz isotopique, et la deuxième extrémité étant reliée à la première chambre et à l'enceinte de mélange pour remplir la première chambre et/ou l'enceinte de mélange ;
- la ligne d'alimentation est reliée à l'enceinte de mélange, à la première chambre, et à la source de gaz respectivement par l'intermédiaire d'une première vanne, d'une deuxième vanne, et d'une troisième vanne ;
- la source de gaz est en outre reliée à un moyen de récupération du gaz considéré, le moyen de récupération pouvant être un piège cryogénique ;
- la ligne d'alimentation est en outre reliée à un moyen de filtrage pour assécher les gaz circulant dans la ligne d'alimentation, le moyen de filtrage pouvant être un tamis moléculaire pour piéger les molécules d'eau des gaz circulant dans la ligne d'alimentation ;
- le moyen de filtrage comprend une entrée et une sortie, l'entrée et la sortie étant respectivement reliées à la ligne d'alimentation par l'intermédiaire d'une quatrième et d'une cinquième vanne, la ligne d'alimentation comprenant en outre une sixième vanne placée entre la quatrième et la cinquième vanne pour court-circuiter la ligne d'alimentation ;
- la ligne d'alimentation comprend en outre une septième vanne interposée au niveau de la deuxième extrémité avant les première et deuxième vannes pour isoler la ligne d'alimentation de l'enceinte de mélange et de la première chambre de l'enceinte de perméation ;
- la ligne d'alimentation est en outre reliée, par l'intermédiaire d'une huitième vanne, à une réserve de gaz qui pourra être une bouteille d'hélium équipée d'un manomètre détendeur ;
- la ligne d'alimentation est en outre reliée, au niveau de la première extrémité, par l'intermédiaire d'une neuvième vanne, à un premier moyen de pompe pour faire le vide dans la ligne d'alimentation ;
- l'enceinte de mélange est en outre reliée à un moyen de régulation de phase d'espèces de l'enceinte de mélange, le moyen de régulation de phase pouvant être un module à effet Pelletier régulant la température d'une solution saline saturée d'eau deutérée ;

- le dispositif comprend en outre un moyen de régulation thermique pour réguler la température de l'enceinte de perméation et l'enceinte de mélange ;
- le moyen d'analyse comprend un analyseur placé dans une enceinte de mesure comprenant une première et une deuxième extrémité, la première extrémité étant reliée à la deuxième chambre, et la deuxième extrémité étant reliée à un deuxième moyen de pompe pour mettre l'enceinte de mesure sous vide ;
- la première extrémité de l'enceinte de mesure est reliée à la deuxième chambre par l'intermédiaire d'une dixième vanne ;
- le deuxième moyen de pompe comprend une pompe primaire et une pompe secondaire placées en série ; le deuxième moyen de pompe est relié à la deuxième extrémité de l'enceinte de mesure par l'intermédiaire d'une onzième vanne ;
- l'analyseur est un spectromètre de masse, le spectromètre de masse pouvant être positionné sur le chemin du flux de gaz entre la deuxième chambre et les pompes.

Enfin, on prévoit selon un exemple ne faisant pas partie de l'invention un procédé d'utilisation d'un tel dispositif de mesure de perméation, caractérisé en ce qu'il comprend les étapes consistant à :

- Mettre en place un film échantillon d'un matériau à mesurer dans l'enceinte de perméation, et fermer toutes les vannes du dispositif ;
- Ouvrir la dizième et la onzième vannes et mettre la deuxième chambre sous vide avec le deuxième moyen de pompe ;
- Mettre en place la solution saline saturée d'eau deutérée dans l'enceinte de mélange et geler la solution avec le module à effet Pelletier ;
- Ouvrir les première, deuxième, quatrième, cinquième, sixième, septième, et neuvième vannes et mettre sous vide la première chambre, la ligne d'alimentation, l'enceinte de mélange, et le moyen de filtrage avec le premier moyen de pompe ;
- Fermer les deuxième, quatrième, cinquième, et neuvième vannes ;
- Ouvrir la troisième vanne pour remplir l'enceinte de mélange avec un gaz isotopique de la source de gaz isotopique ;
- Fermer la première vanne et récupérer le gaz isotopique de la ligne d'alimentation avec le moyen de récupération ;
- Fermer les troisième, sixième, et septième vannes et laisser le mélange s'équilibrer ;
- Ouvrir les première et deuxième vannes et calculer simultanément la perméation du matériau à chacun des gaz cibles avec le moyen d'analyse.

Ce procédé d'utilisation peut en outre comprendre les étapes consistant à :

- Geler la solution saline avec le module à effet Pelletier ;
- Fermer la sixième vanne et ouvrir les troisième, quatrième, cinquième, et septième vannes, et assécher les gaz avec le moyen de filtrage ;
- Récupérer les gaz asséchés avec le moyen de récupération et fermer la troisième vanne.

DESCRIPTION DES FIGURES

D'autres caractéristiques et avantages de l'invention ressortiront encore de la description qui suit, laquelle est purement illustrative et non limitative et doit être lue en regard des dessins annexés, sur lesquels :

- la figure 1 est un schéma de principe illustrant le dispositif de mesure de perméation pouvant être utilisé pour mettre en oeuvre le procédé de l'invention ;
- la figure 2 est une vue en coupe du mécanisme de fixation d'un film dans le dispositif de perméation ;
- la figure 3 est une vue de dessus du mécanisme de la figure 2 ;
- la figure 4 représente des courbes illustrant un exemple de mesure combinée.

DESCRIPTION DETAILLEE DE L'INVENTION

Le processus de perméation d'un gaz à travers un matériau M repose sur les différences de pressions partielles de ce gaz, appelé aussi perméant, de part et d'autre du matériau M, échantillonné de manière générale sous la forme d'un film F.

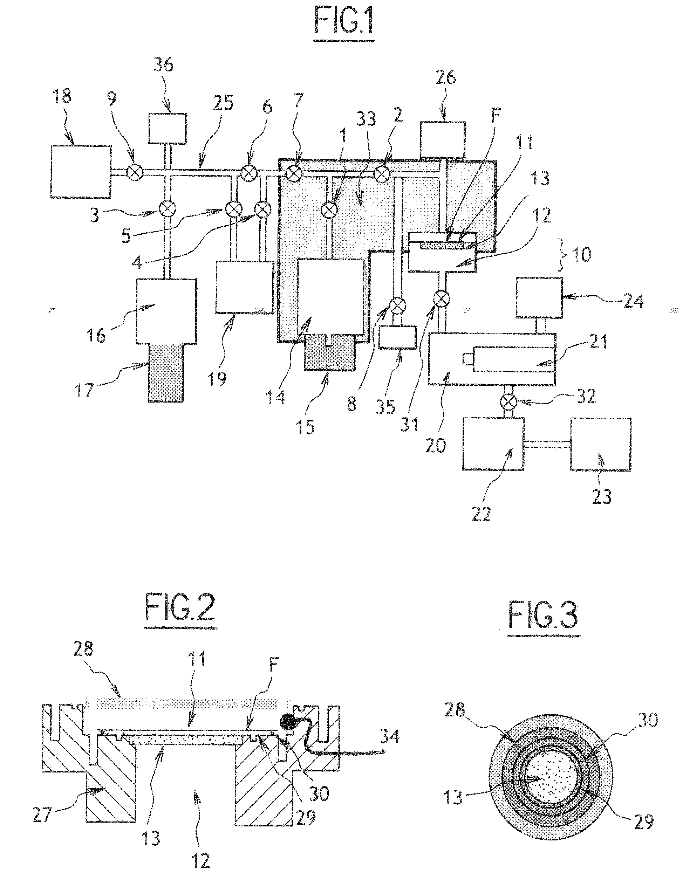

Le perméamètre, dont le schéma de principe est illustré à la figure 1, comprend une enceinte de perméation 10 qui comprend une première chambre 11 et une deuxième chambre 12 séparées par le film F de matériau M. Pour l'étude des différences de pressions partielles du perméant entre la première chambre 11, située en amont dans le dispositif, et la deuxième chambre 12, située en aval dans le dispositif, il est important d'assurer une pression partielle avale nulle, ou négligeable, par rapport à la pression amont. Une atmosphère contrôlée, avec une pression amont déterminée en perméant(s), est réalisée au dessus du film à tester. En dessous du film, la deuxième chambre 12, dans laquelle un vide poussé est maintenu, permet d'assurer la condition de pression avale nulle, ou négligeable, au regard de la pression amont.

Afin d'éviter une déformation excessive du film F, l'enceinte de perméation 10 comprend un moyen support 13, tel qu'un fritté métallique poreux. Le film F repose ainsi sur ce moyen de support 13 qui est disposé entre la première chambre 11 et la deuxième chambre 12, et est formé de manière à permettre une transmission des gaz.

Les figures 2 et 3 illustrent un mécanisme de fixation qui peut être prévu à l'intérieur de l'enceinte de perméation pour fixer un film échantillon F dans l'enceinte de perméation 10.

Le mécanisme de fixation comprend une armature 27 ayant sensiblement une symétrie de révolution et présentant une ouverture traversante selon l'axe de révolution. L'armature 27 est formée de manière à pouvoir recevoir le fritté métallique 13 pour séparer la première chambre 11 de la deuxième chambre 12. Le fritté métallique 13 pourra par exemple avoir une forme de disque, ce disque étant supporté par une partie coudée prévue sur la périphérie interne de l'armature 27. Le film échantillon F de matériau M est ensuite posé sur ce fritté métallique 13. On prévoira par exemple un disque échantillon ayant une surface supérieure à la surface du fritté métallique 13 de façon à pouvoir recouvrir entièrement le fritté métallique 13. En pratique, la dimension du film échantillon F est de l'ordre de 12 cm$^2$.

En outre, pour assurer une parfaite étanchéité entre la première chambre 11 et la deuxième chambre 12, on prévoit des joints disposés entre le fritté métallique 13 et le film échantillon F, sur la périphérie du film échantillon F. Un anneau de serrage 28 vient en appui contre le film F pour compresser les joints et assurer l'étanchéité entre la première chambre 11 et la deuxième chambre 12. On utilisera par exemple un joint torique 29 et un joint indium 30.

En aval du film F, connecté à la deuxième chambre 12, on prévoit un moyen d'analyse adapté pour suivre la pression partielle avale en perméant(s). Le moyen d'analyse du dispositif de mesure comprend une enceinte de mesure 20 raccordée à la deuxième chambre 12 par l'intermédiaire d'une vanne 31 permettant d'isoler, si nécessaire, l'enceinte de mesure 20 de la deuxième chambre 12. Cette enceinte de mesure 20 est mise sous vide par un système de pompage comprenant une pompe primaire 23 et une pompe secondaire 22, qui sont préférentiellement placées en série l'une par rapport à l'autre. L'enceinte de mesure et le système de pompage sont reliés par l'intermédiaire d'une vanne 32.

En outre, le moyen d'analyse comprend un analyseur 21 de gaz résiduels placés dans l'enceinte de mesure 20 sous vide, et disposé de manière à pouvoir analyser avec la meilleure sensibilité possible les gaz provenant de la deuxième chambre 12 avant d'être aspirés par les pompes 22 et 23. L'analyseur 21 pourra par exemple être un spectromètre de masse placé dans une enceinte de mesure 20 mise sous vide par une pompe primaire sèche 23 et une turbo pompe 22 placées en série. Pour une meilleure sensibilité, le spectromètre est positionné sur le chemin du flux de gaz entre la deuxième chambre 12 et les pompes 22 et 23. Un capteur de pression 24 peut également être prévu pour mesurer la pression totale régnant au sein de l'enceinte de mesure 20.

Le spectromètre de masse 21 utilisé ne peut fonctionner qu'à basses pressions, et nécessite donc une enceinte de mesure 20 sous un vide secondaire (de l'ordre de 10$^{-4}$ torr maximum). De ce fait, on pompe en permanence l'enceinte de mesure 20 avec les pompes 22 et 23 pendant le transfert des gaz par perméation à travers le film échantillon F. Ainsi, la pression partielle de chacun des gaz ayant traversé l'échantillon augmente jusqu'à se stabiliser lorsque le flux de perméation devient égal au flux pompé. Le spectromètre de masse 21 délivre un signal qui est un courant résultant de l'ionisation des atomes d'une espèce mesurée, qui est proportionnel à la pression partielle de cette espèce. Le pompage étant efficace et la perméation des échantillons faible, la pression de stabilisation est également très faible de sorte que l'on peut considérer que la pression sur la face avale du film échantillon F reste quasi nulle au cours du temps. Le flux d'un perméant à travers le film échantillon F est donc proportionnel à la pression partielle du perméant suivi quand le régime permanent est atteint. Pour des flux de perméation extrêmement bas, la fermeture de la vanne 32 permettra une mesure cumulative dans la limite de pression totale fixée par le moyen d'analyse 21 soit de l'ordre de 10$^{-4}$ torr ce qui permettra de respecter le critère de pression avale négligeable au regard de la pression amont.

A partir du flux de perméation ainsi calculé, on pourra en déduire la perméabilité du matériau M au gaz considéré en considérant l'épaisseur e du film échantillon F.

En outre, l'examen des flux de perméation cumulés sur l'ensemble de l'expérience permet d'obtenir le coefficient de diffusion selon la méthode connue du « time-lag ». Cette méthode consiste à tracer le cumul du flux de perméation d'un gaz en fonction du temps, et à réaliser une extrapolation de la régression linéaire à cumul nul à partir des valeurs correspondant au régime permanent. Le temps $T_L$ obtenu est directement lié au coefficient de diffusion D par la formule suivante :

$$D = \frac{e^2}{6.T_L}$$

La sensibilité des mesures est déterminée par le niveau de pollution résiduelle de la masse suivie par spectrométrie dans l'enceinte de mesure 20 sous vide. L'eau $H_2O$ et l'oxygène $O_2$, ayant respectivement des nombres de masse de 18 et 32, sont naturellement parmi les polluants les plus importants, et sont pourtant les espèces les plus importantes dans l'étude de la perméation à travers un matériau M. Pour s'affranchir de ce problème de pollution résiduelle, que ce soit pour l'eau ou pour l'oxygène, mais aussi pour les autres gaz à étudier, on effectue les mesures à l'aide de gaz isotopiques, comme par exemple le deutérium ($D_2O$) pour la vapeur d'eau, avec un nombre de masse de 20, et l'oxygène 18 ($^{18}O_2$) pour l'oxygène, avec un nombre de masse de 36.

Comme on l'a précisé plus haut, il est nécessaire, pour connaître la perméabilité d'un matériau M à un gaz particulier, d'atteindre un régime permanent pendant l'analyse. Or, pour des gaz tels que la vapeur d'eau ou l'oxygène, ce régime permanent, pour être atteint, peut nécessiter des temps longs lorsque les propriétés barrières du matériau M sont importantes. Ainsi, la caractérisation complète d'un matériau M quant à sa perméation par rapport à un ensemble de gaz considérés, et notamment la perméation de l'oxygène et la vapeur d'eau, peut prendre plusieurs semaines.

Dans un processus de sélection d'un matériau M pour une utilisation ultérieure en tant que matériau pour de l'encapsulation par exemple, une étape du procédé selon l'invention consiste à réaliser une présélection du matériau en mesurant la perméation de l'hélium He à travers le matériau considéré. En effet, l'hélium interagissant peu avec le matériau et ayant un faible rayon moléculaire, il possède des temps de diffusions courts, ce qui réduit d'autant les temps de mesure puisque le régime permanent est atteint beaucoup plus rapidement, généralement en quelques minutes, voire quelques heures pour les matériaux les plus performants en termes de propriétés barrières. Mêmes si les mécanismes de perméation de l'hélium diffèrent très sensiblement de ceux de la vapeur d'eau ou de l'oxygène, les mesures hélium définissent grossièrement les propriétés barrières du matériau et affinent la sélection des matériaux nécessitant des mesures de perméation à la vapeur d'eau et/ou à l'oxygène. Par exemple, si l'on cherche des matériaux présentant des perméabilités aux gaz très faibles (comme c'est le cas pour l'encapsulation où les flux respectifs acceptables en vapeur d'eau et en oxygène s'établissent à $10^{-6}$ g/m$^2$.j et $10^{-3}$ g/m$^2$.j), alors il n'est pas nécessaire de faire les mesures complémentaires quand la perméabilité du matériau à l'hélium est importante. La mesure de la perméation de l'hélium permet donc une présélection qui pourra éviter de réaliser les mesures ultérieures de la perméabilité du matériau à la vapeur d'eau et à l'oxygène qui sont très longues. Cette solution comporte néanmoins l'inconvénient de ne pouvoir être applicable que lors d'un processus de sélection de matériau, et ne permettra pas de réduire les temps de mesure dans les cas où la caractérisation complète du matériau à étudier, et notamment en termes de perméation de l'eau et de l'oxygène, est à réaliser. Pour ce faire, on utilisera par exemple une réserve de gaz 35 qui pourra être une bouteille d'hélium équipée d'un manomètre détendeur, cette réserve de gaz 35 étant reliée à la ligne d'alimentation 25 par l'intermédiaire d'une huitième vanne. L'injection d'hélium dans la ligne de gaz 25 se fera directement par la réserve 35 après ouverture de la vanne 8 et fermeture de la vanne 2. La mesure de la pression injectée se fera à l'aide du moyen de contrôle de pression 26.

Une solution complémentaire consiste à réaliser les mesures de perméation de différents gaz de manière simultanée. Pour ce faire, il est nécessaire d'injecter dans la première chambre 11 de l'enceinte de perméation 10 une atmosphère comportant un mélange de plusieurs gaz isotopiques, qui pourront diffuser de manière simultanée à travers le matériau M à étudier. Néanmoins, la réalisation d'une telle atmosphère comportant plusieurs gaz différents nécessite un moyen d'alimentation en gaz particulier que l'on va maintenant décrire en détail.

Le moyen d'alimentation en gaz du dispositif de mesure comprend une ligne principale d'alimentation en gaz 25 par l'intermédiaire de laquelle un mélange de gaz isotopiques va pouvoir être formé, ce mélange de gaz isotopiques étant ensuite injecté dans la première chambre 11 de l'enceinte de perméation 10.

En effet, la ligne d'alimentation en gaz 25 comprend une première et une deuxième extrémité, la deuxième extrémité étant reliée à la première chambre 11 de l'enceinte de perméation 10 par l'intermédiaire d'une vanne 2 qui peut être fermée ou ouverte selon que l'on veut isoler ou non la première chambre 11 de la ligne d'alimentation en gaz 25.

A cette deuxième extrémité, est également reliée une enceinte de mélange 14 par l'intermédiaire d'une vanne 1 permettant également d'isoler ou non l'enceinte de mélange 14 de la ligne d'alimentation en gaz 25. C'est dans cette enceinte de mélange 14 qu'un mélange de gaz isotopiques sera formé avant d'être injecté dans la première chambre 11.

Un module à effet Pelletier 15 est en outre couplé à cette enceinte de mélange 14 pour pouvoir former le mélange de gaz isotopiques. Ce module à effet Pelletier 15 joue le rôle d'un moyen de régulation de phase des espèces présentes dans l'enceinte de mélange 14, utile notamment durant les étapes de pompage nécessaires à l'établissement du mélange. Comme on cherche à obtenir un mélange de gaz isotopiques, on pourra par exemple utiliser, pour la mesure de la perméation de la vapeur d'eau, une solution saline d'eau deutérée placée dans un godet au sein de l'enceinte de mélange 14. L'utilisation d'un module à effet Pelletier 15 permettra un contrôle accru de la température de la solution saline en eau deutérée du godet, et du même coup de la transformation de phase (solide ou liquide) de l'eau deutérée. En outre, la nature du sel utilisé dans la solution saline permet d'obtenir une gamme complète d'hygrométrie relative adaptée au mélange désiré. Cela permettra de contrôler la pression partielle amont en vapeur d'eau, cet élément influant bien entendu les valeurs de perméation en vapeur d'eau mais également celles d'autres perméants, comme l'oxygène par exemple, en raison d'effets divers sur le matériau.

Indépendamment du moyen de régulation de phase 15, on peut prévoir un moyen de régulation thermique 33 couplé à l'enceinte de mélange 14 ainsi qu'à l'enceinte de perméation 10 permettant, durant la mesure, de thermostater l'ensemble amont, dont le film F de matériau M, pour étudier notamment les mécanismes de perméation en fonction de la température. En effet, la température influe grandement sur les propriétés barrières du matériau. A ce titre, une sonde thermique 34 sera placée au plus prés du film F.

La première extrémité de la ligne d'alimentation 25 est quant à elle reliée à une source de gaz 16, qui pourra par exemple être une source de gaz isotopique comme l'oxygène 17 ($^{17}O_2$) ou l'oxygène 18 ($^{18}O_2$). Cette source de gaz 16 est reliée à la première extrémité de la ligne d'alimentation en gaz 25 par l'intermédiaire d'une vanne 3 de manière à pouvoir injecter un gaz dans la ligne d'alimentation 25, et donc dans l'enceinte de mélange 14 ou directement dans la première chambre 11. La maîtrise de la pression injectée sera réalisée avec le moyen de mesure de pression 36.

Cette source de gaz 16 pourra être couplée à un moyen de récupération 17 adapté au gaz considéré et permettant de piéger le gaz correspondant présent dans la ligne d'alimentation en gaz 25. On utilisera par exemple un piège à froid tel qu'un piège cryogénique.

Il est à noter que d'autres sources de gaz (non représentées) peuvent être reliées à la première extrémité de la ligne d'alimentation 25. On pourra par exemple prévoir des sources pour d'autres gaz dont on cherche à déterminer directement la perméation à travers le matériau M, mais aussi toute autre source de gaz, isotopique ou non, permettant de réaliser le mélange destiné à être injecté dans la première chambre 11.

Un moyen de pompe 18 est également relié à la première extrémité de la ligne d'alimentation 25 par l'intermédiaire d'une vanne 8. Ce moyen de pompe 18 permettra, le moment voulu, de faire le vide dans la ligne d'alimentation en gaz 25. Ce sera par exemple le cas avant d'injecter le mélange de gaz isotopiques dans la première chambre 11, mais également quand on voudra analyser de nouveaux matériaux M. On utilisera par exemple un pompe primaire sèche.

Enfin, on peut également prévoir sur la ligne d'alimentation 25 un moyen de filtrage 19 prévu pour assécher les gaz circulant dans la ligne d'alimentation 25, notamment à l'issue des mesures réalisées sur le matériau M. Ainsi, ce moyen de filtrage 19 comprend une entrée et une sortie reliées à la ligne d'alimentation 25 respectivement par une vanne 4 et une vanne 5. En outre, une vanne 6 est placée sur la ligne d'alimentation 25 entre la vanne 4 et la vanne 5 pour court-circuiter la ligne d'alimentation en gaz si nécessaire. Le moyen de filtrage 19 pourra par exemple être un tamis moléculaire permettant de piéger les molécules d'eau des gaz circulant dans la ligne d'alimentation 25.

La description détaillée qui suit d'une mesure combinée de la perméation de la vapeur d'eau et de l'oxygène à travers un matériau M à analyser permet d'illustrer le fonctionnement du dispositif de mesure, et plus particulièrement du moyen d'alimentation en gaz particulier qui permet une injection d'un mélange de gaz isotopiques dans la première chambre 11.

Une étape préalable à la mesure, et nécessaire à toute analyse d'un matériau M, est la mise en place du film échantillon F de matériau M à analyser dans l'enceinte de perméation 10. Pour ce faire, on va d'abord fermer l'ensemble des vannes du dispositif de mesure. On place ensuite le film échantillon F à analyser sur le fritté métallique 13 et on le fixe dans l'enceinte de perméation 10 avec l'anneau de serrage 28 du mécanisme de fixation. Une fois le film échantillon F fixé dans l'enceinte de perméation 10, on peut fermer cette enceinte.

Il convient alors de créer un vide secondaire en aval du film échantillon F à l'aide des pompes primaire 23 et secondaire 22 du moyen d'analyse. On peut prévoir, lors des changements de films échantillons F, la fermeture de la vanne 31, ce qui permet de maintenir le vide secondaire dans l'enceinte 20 et de limiter la contamination à la chambre 12. Ce système permet de limiter la pollution de la chambre d'analyse, même si, avant de recréer un vide primaire dans la chambre 12, il convient de repasser en vide primaire dans l'enceinte 20 afin de ne pas échauffer la pompe secondaire. Ainsi, pour créer un vide primaire (de l'ordre de $10^{-3}$ torr) en aval du film échantillon F, il suffit de couper la pompe secondaire 22 et d'ouvrir la vanne 31. Une fois le vide primaire atteint dans l'enceinte 20 et la chambre 12, la pompe secondaire 22 permet d'atteindre un vide secondaire de l'ordre de $10^{-7}$ torr dans l'ensemble 20 et 12.

Pendant que le vide secondaire est ainsi réalisé en aval du film échantillon F, on peut former le mélange de gaz isotopiques au sein de l'enceinte de mélange 14 avant de l'injecter dans la première chambre 11 de l'enceinte de perméation 10. Dans notre exemple, on réalise une atmosphère comprenant un mélange de vapeur d'eau deutérée et d'oxygène 18, de manière à pouvoir ensuite réaliser simultanément des analyses de la perméation de l'eau deutérée et de l'oxygène 18 à travers le film échantillon F.

La première étape consiste à mettre en place une solution saline saturée d'eau deutérée dans un godet placé dans l'enceinte de mélange 14. Cette solution est ensuite gelée avec le module à effet Pelletier 15 qui est couplé à l'enceinte de mélange 14. On ouvre ensuite les vannes 1, 2, 4, 5, 6, 7 et 9 et on active la pompe primaire sèche 18 pour mettre sous vide la ligne d'alimentation en gaz 25 mais également l'enceinte de mélange 14, le moyen de filtrage 19, et la première chambre 11.

On ferme ensuite les vannes 2, 4, 5 et 9 et on ouvre la vanne 3 pour ouvrir la source de gaz, qui est en l'espèce une source en oxygène 18, de manière à remplir l'enceinte de mélange 14 avec une certaine quantité d'oxygène 18. La pression injectée est contrôlée avec le moyen de mesure de pression 36.

On ferme ensuite la vanne 1 et on active le piège cryogénique 17 couplé à la source de gaz 16 de manière à récupérer l'oxygène 18 restant dans la ligne d'alimentation 25 pour le piéger dans la source 16 en oxygène 18.

On ferme ensuite les vannes 3, 6, et 7 et on laisse le mélange s'équilibrer au sein de l'enceinte de mélange 14 après avoir dégelé le godet d'eau avec le module à effet Pelletier 15. On pourra par exemple laisser le mélange eau deutérée/oxygène 18 s'équilibrer pendant environ 24 heures.

Une fois que le mélange de gaz isotopiques est formé et l'enceinte de mélange 14 et la chambre de perméation 10 ont été thermostatées à la température voulue à l'aide du moyen de régulation thermique 33, on peut commencer les mesures. Pour ce faire, on ouvre les vannes 1 et 2 de manière à ce que le mélange de l'enceinte de mélange 14 emplisse la première chambre 11 et que le processus de perméation à travers le film échantillon F commence. On utilise un capteur de pression 26 situé sur la première chambre 11 pour contrôler la pression totale du mélange de gaz isotopiques dans la première chambre 11.

Les mesures simultanées des différents flux de perméation de l'eau deutérée et de l'oxygène 18 sont réalisées par le spectromètre 21.

Comme expliqué ci dessus, le flux de perméation augmente jusqu'à se stabiliser et on peut, une fois le régime permanent atteint, déterminer la pression partielle de chacun des gaz isotopiques suivis.

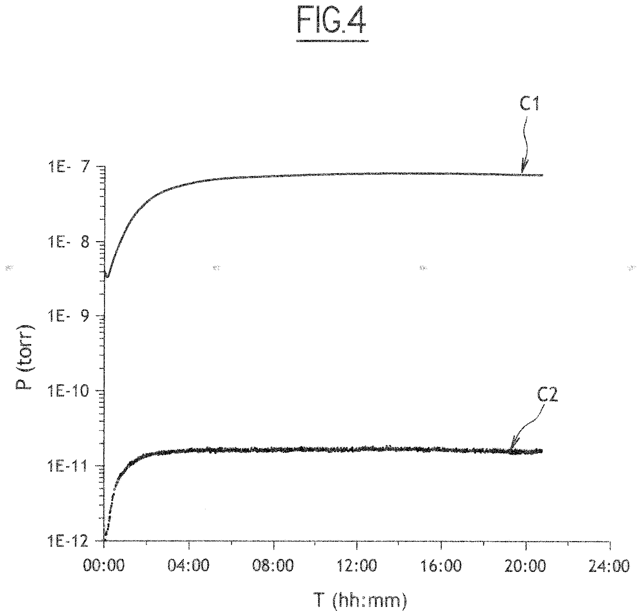

La figure 4 présente un exemple de courbes d'évolution de la pression partielle (en torr) en eau deutérée (courbe C1) et en oxygène 18 (courbe C2) en fonction du temps (en hh:mm). Ces courbes de résultats sont issues d'une mesure combinée eau deutérée/oxygène 18 sur un film d'un matériau polymère revêtu d'une couche inorganique avec une atmosphère comprenant un mélange de gaz isotopiques issu d'une solution saline saturée en NaCl fixant un taux d'hygrométrie de 80% et une quantité d'oxygène 18 correspondant à une pression de 700 torr. L'ensemble est thermostaté à 23°C. Le dispositif avait été au préalable étalonné avec des films de référence de perméabilité connue.

Une fois les flux de l'eau deutérée et de l'oxygène 18 à travers le matériau M stabilisés, il convient de purger la première chambre 11 du mélange de gaz isotopiques. Pour ce faire, on pourra prévoir un moyen de purge quelconque au niveau de la première chambre 11 de sorte que le mélange de gaz isotopiques soit par exemple rejeté dans l'atmosphère environnant.

Une autre solution, qui est préférée mais pas obligatoire, consiste à récupérer le mélange de gaz isotopiques et à isoler chacun des gaz le constituant. Cette récupération sera notamment possible grâce au moyen de filtrage 19.

Ainsi, pour récupérer le mélange de gaz isotopiques, on procédera d'abord à la congélation de la solution de l'enceinte de mélange 14 avec le module à effet Pelletier 15.

On ferme ensuite la vanne 6 et on ouvre les vannes 3, 4, 5, et 7 de façon à faire passer le mélange de gaz isotopiques à travers le moyen de filtrage 19 qui pourra par exemple être un tamis moléculaire destiné à retirer les molécules d'eau présentes dans le mélange.

Le mélange de gaz ayant été asséché, il ne reste, dans ce cas, que de l'oxygène 18 qui peut être piégé dans la source de gaz 16 grâce au piège cryogénique 17 prévu à cet effet, avant de fermer la vanne 3.

Ce processus de récupération des gaz permet non seulement une économie des gaz utilisés mais permet également de réduire la pollution due au rejet des gaz dans l'environnement.

L'activation de la pompe primaire sèche 18 permettra éventuellement de purger complètement la ligne d'alimentation en gaz 25, le moyen de filtrage 19, et la première chambre 11.

Comme on l'a dit précédemment, ces mesures combinées eau deutérée/oxygène 18 sont dans le procédé de l'invention conditionnées par une analyse préalable de la perméation de l'hélium à travers le film échantillon F du matériau M à analyser. Dans le cas où les analyses de perméation de l'hélium à travers le matériau M révèlent que les propriétés du matériau M ne sont pas satisfaisantes, il sera possible de ne pas analyser le matériau plus en détail, notamment en ce qui concerne la perméabilité du matériau M à l'eau et à l'oxygène.

Si les mesures de perméation de hélium sont au contraire satisfaisantes, le dispositif de mesure permet d'effectuer les analyses ultérieures de la perméation de l'eau et de l'oxygène sur le même film échantillon M, sans avoir à modifier la configuration du dispositif de mesure.

En effet, il suffit de prévoir une source en hélium reliée à la première extrémité de la ligne d'alimentation en gaz 25 par l'intermédiaire d'une vanne. Cette source permettra ainsi de créer une atmosphère contrôlée en hélium au sein de la première chambre pour mesurer la perméation en hélium du film échantillon F considéré. Il convient ensuite de récupérer l'hélium restant dans la première chambre 11 à l'issue des analyses de la perméation de l'hélium et de purger avec la pompe primaire sèche 18 la ligne d'alimentation en gaz 25 et la première chambre 11. Les mesures combinées en eau deutérée et en oxygène 18, et notamment la phase préalable de formation du mélange de gaz isotopiques pourra donc commencer.

Le dispositif de mesure de perméation décrit présente donc l'avantage de pouvoir réaliser l'ensemble des mesures de perméation sur un même film échantillon F de dimensions modestes au regard des appareils commerciaux sans créer d'écarts trop importants en ce qui concerne les conditions expérimentales des analyses de perméation successives.

En outre, on peut prévoir, à l'issue des analyses combinées de perméation en eau deutérée et en oxygène 18, de procéder à une nouvelle analyse de la perméation de hélium à travers le film échantillon F de manière à pouvoir corréler les résultats et ainsi prendre en compte les changements structurels qu'aurait pu subir le film F au cours des analyses successives.

De tels procédé et dispositif pour des mesures simultanées de perméation de gaz isotopiques à travers un matériau sont particulièrement intéressants dans les cas de la sélection de matériaux pour l'encapsulation de cellules solaires organiques par exemple. En effet, le dispositif permet des mesures selon une sensibilité accrue, de l'ordre de $10^{-5}$ g/m$^2$.j pour la vapeur d'eau. Bien sûr, l'utilisation des procédé et dispositif de mesure décrits n'est pas limitée à l'encapsulation de cellules solaires organiques et pourra s'étendre à toute application qui nécessite des mesures de flux surfaciques avec des seuils très faibles.

**Revendications**

1. Procédé de sélection d'un matériau (M) par mesure de perméation de gaz à travers ledit matériau (M) utilisant une enceinte de perméation (10) comprenant une première (11) et une deuxième chambre (12), la première chambre (11) étant séparée de la deuxième (12) chambre par le matériau (M), ledit procédé comprenant une étape préliminaire de présélection consistant à mesurer la perméation de l'hélium à travers ledit matériau (M) et à exclure le matériau (M) de l'étape ultérieure de mesure de perméation de gaz si la perméation du matériau (M) à l'hélium est supérieure à un seuil déterminé, dans lequel :.

    - l'étape préliminaire de présélection comprend les étapes consistant à:

        ◦ remplir avec de l'hélium la première chambre (11) de l'enceinte de perméation (10) ; et
        ◦ calculer la perméation du matériau (M) à l'hélium ;

    - et l'étape de mesure de perméation de gaz comprend les étapes consistant à :

        ◦ Former un mélange comprenant plusieurs gaz isotopiques dans une enceinte de mélange (14) distincte de la première chambre (11) de l'enceinte de perméation (10), chaque gaz isotopique correspondant, avec un nombre de masse différent, à un gaz cible dont on cherche à connaître la perméation à travers le matériau (M) ;
        ◦ Remplir avec le mélange de gaz la première chambre (11) ;
        ◦ Analyser simultanément les différents gaz du mélange de gaz ayant traversé le matériau (M) par perméation et étant présents dans la deuxième chambre (12), pour calculer simultanément la perméation du matériau (M) à chacun des gaz cibles correspondants.

2. Procédé selon la revendication 1, dans lequel l'étape de mesure de perméation de gaz est prévue pour déterminer la perméation du matériau (M) à la vapeur d'eau, la mesure de perméation étant effectuée en utilisant du deutérium ($D_2O$).

3. Procédé selon l'une quelconque des revendications 1 et 2, dans lequel l'étape de mesure de perméation de gaz est prévue pour déterminer la perméation du matériau (M) à l'oxygène ($O_2$), la mesure de perméation étant effectuée en utilisant de l'oxygène 18 ($^{18}O_2$).

4. Procédé selon l'une quelconque des revendications 1 et 2, dans lequel l'étape de mesure de perméation de gaz est prévue pour déterminer la perméation du matériau (M) à l'oxygène ($O_2$), la mesure de perméation étant effectuée en utilisant de l'oxygène 17 ($^{17}O_2$).

5. Procédé selon l'une quelconque des revendications 1 à 4, dans lequel, à l'issue des analyses de la perméation de l'hélium, on récupère l'hélium restant dans la première chambre (11) et on purge avec une pompe primaire sèche (18) la première chambre (11) ainsi qu'une ligne d'alimentation en gaz (25) de ladite première chambre (11).

6. Procédé selon l'une quelconque des revendications 1 à 5, dans lequel, après l'étape de mesure de perméation, on procède à une nouvelle analyse de la perméation de l'hélium à travers le matériau (M) et on corrèle cette analyse aux résultats des analyses précédentes afin de déterminer d'éventuels changements structurels du matériau (M) au cours des analyses successives.

**Patentansprüche**

1. Verfahren zur Auswahl eines Materials (M) durch Durchlässigkeitsmessung von Gas durch das Material (M) mittels Verwendung eines Durchlässigkeitsbehälters (10), umfassend eine erste (11) und eine zweite Kammer (12), wobei die erste Kammer (11) von der zweiten Kammer (12) durch das Material (M) getrennt ist, wobei das Verfahren einen vorherigen Schritt der Vorauswahl umfasst, der darin besteht, die Durchlässigkeit von Helium durch das Material (M) zu messen und das Material (M) des vorherigen Schritts der Durchlässigkeitsmessung von Gas auszuschließen, wenn die Durchlässigkeit des Materials (M) für Helium größer als ein bestimmter Grenzwert ist, wobei

    - der vorherige Schritt der Vorauswahl die Schritte umfasst, die darin bestehen:

        ◦ Füllen der ersten Kammer (11) des Durchlässigkeitsbehälters (10) mit Helium; und
        ◦ Berechnen der Durchlässigkeit des Materials (M) für Helium;

    - und der Schritt des Messens der Gasdurchlässigkeit die Schritte umfasst, die darin bestehen:

        ◦ Bilden eines Gemischs, das mehrere isotopische Gase in einem Mischbehälter (14) umfasst, der sich von der ersten Kammer (11) des Durchlässigkeitsbehälters (10) unterscheidet, wobei jedes isotopische Gas mit einer unterschiedlichen Massenzahl einem Zielgas entspricht, dessen Durchlässigkeit durch das Material (M) ermittelt werden soll;

∘ Füllen, der ersten Kammer (11), mit dem Gasgemisch;

∘ gleichzeitiges Analysieren der unterschiedlichen Gase des Gasgemischs, die das Material (M) mittels Durchlässigkeit durchquert haben und in der zweiten Kammer (12) vorhanden sind, um gleichzeitig die Durchlässigkeit des Materials (M) für jedes der entsprechenden Zielgase zu berechnen.

2. Verfahren nach Anspruch 1, wobei der Schritt der Durchlässigkeitsmessung von Gas vorgesehen ist, um die Durchlässigkeit des Materials (M) für Wasserdampf zu bestimmen, wobei die Durchlässigkeitsmessung unter Benutzung von Deuterium ($D_2O$) durchgeführt wird.

3. Verfahren nach einem der Ansprüche 1 und 2, wobei der Schritt der Durchlässigkeitsmessung von Gas vorgesehen ist, um die Durchlässigkeit des Materials (M) für Sauerstoff ($O_2$) zu bestimmen, wobei die Durchlässigkeitsmessung unter Benutzung von Sauerstoff 18 ($^{18}O_2$) durchgeführt wird.

4. Verfahren nach einem der Ansprüche 1 und 2, wobei der Schritt der Durchlässigkeitsmessung von Gas vorgesehen ist, um die Durchlässigkeit des Materials (M) für Sauerstoff ($O_2$) zu bestimmen, wobei die Durchlässigkeitsmessung unter Benutzung von Sauerstoff 17 ($^{17}O_2$) durchgeführt wird.

5. Verfahren nach einem der Ansprüche 1 bis 4, wobei, im Ergebnis der Analysen der Durchlässigkeit von Helium, das in der ersten Kammer (11) verbliebene Helium zurückgewonnen wird und die erste Kammer (11) mit einer primären Trockenpumpe (18) sowie eine Gasversorgungsleitung (25) der ersten Kammer (11) gereinigt werden.

6. Verfahren nach einem der Ansprüche 1 bis 5, wobei, nach dem Schritt der Durchlässigkeitsmessung, eine neue Analyse der Durchlässigkeit von Helium durch das Material (M) durchgeführt wird und diese Analyse mit den Ergebnissen der vorangegangenen Analysen korreliert werden, um eventuelle strukturelle Veränderungen des Materials (M) während der aufeinanderfolgenden Analysen zu bestimmen.

**Claims**

1. Method for selecting a material (M) by measuring gas permeation through said material (M) using a permeation enclosure (10) comprising a first (11) and a second chamber (12), the first chamber (11) being separated from the second (12) chamber by the material (M), said method comprising a preliminary preselection step consisting in measuring the permeation of helium through said material (M) and excluding the material (M) from the subsequent gas permeation measurement step if the permeation of the material (M) to helium is greater than a specified threshold, wherein

- the preliminary preselection step comprises the steps consisting in:

∘ filling the first chamber (11) of the permeation enclosure (10) with helium; and
∘ calculating the permeation of the material (M) to helium;

- and the gas permeation measurement step comprises the steps consisting in:

∘ forming a mixture comprising several isotopic gases in a mixing enclosure (14) separate from the first chamber (11) of the permeation enclosure (10), each isotopic gas corresponding, with a different mass number, to a target gas whose permeation through the material (M) is sought;
∘ filling the first chamber (11) with the gas mixture;
∘ simultaneously analyzing the different gases of the gas mixture that have crossed the material (M) by permeation and are present in the second chamber (12), to simultaneously calculate the permeation of the material (M) to each of the corresponding target gases.

2. Method according to claim 1, wherein the gas permeation measurement step is provided for determining the permeation of the material (M) to water vapor, the permeation measurement being performed using deuterium ($D_2O$).

3. Method according to any one of claims 1 and 2, wherein the gas permeation measurement step is provided for determining the permeation of the material (M) to oxygen ($O_2$), the permeation measurement being performed using oxygen 18 ($^{18}O_2$).

4. Method according to any one of claims 1 and 2, wherein the gas permeation measurement step is provided for determining the permeation of the ma-

terial (M) to oxygen (O$_2$), the permeation measurement being performed using oxygen 17 ($^{17}$O$_2$).

5. Method according to any one of claims 1 to 4, wherein, following helium permeation analyses, the helium remaining in the first chamber (11) is recovered and the first chamber (11) and a gas supply line (25) of said first chamber (11) are purged with a dry primary pump (18).

6. Method according to any one of claims 1 to 5, wherein, after the permeation measurement step, a new analysis of helium permeation through the material (M) is performed and this analysis is correlated with the results of previous analyses in order to determine possible structural changes of the material (M) during successive analyses.

# FIG.1

# FIG.2

# FIG.3

## FIG.4